(19) Europäisches Patentamt
European Patent Office
Office européen des brevets

(11) **EP 2 189 157 A1**

(12) **EUROPEAN PATENT APPLICATION**
published in accordance with Art. 153(4) EPC


(43) Date of publication:
**26.05.2010 Bulletin 2010/21**

(21) Application number: **08829949.0**

(22) Date of filing: **08.09.2008**

(51) Int Cl.:
***A61K 31/335*** (2006.01) ***A61K 47/02*** (2006.01)
***A61K 47/16*** (2006.01) ***A61K 47/32*** (2006.01)
***A61P 27/02*** (2006.01) ***A61P 27/14*** (2006.01)

(86) International application number:
**PCT/JP2008/066185**

(87) International publication number:
**WO 2009/031682 (12.03.2009 Gazette 2009/11)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **06.09.2007 JP 2007231302**
**15.04.2008 JP 2008106245**



(71) Applicant: **Kyowa Hakko Kirin Co., Ltd.**
**Tokyo 100-8185 (JP)**

(72) Inventors:
- **NAKAKURA, Masashi**
**2548, Ohaza-Fujimagari, Ube-shi, Yamaguchi 755-8501 (JP)**
- **ONO, Eikichi**
**1188, Shimotogari, Nagaizumi-cho, Sunto-gun, Shizouka 411-8731 (JP)**
- **MURAI, Kouji**
**Tokyo 100-8185 (JP)**
- **TAMURA, Tadafumi**
**Shizouka 411-8731 (JP)**

(74) Representative: **Vossius & Partner**
**Siebertstraße 4**
**81675 München (DE)**


(54) **EYE DROP COMPRISING DIBENZO[b,e]OXEPIN DERIVATIVE**


(57) The present invention provides an eye drop comprising: a dibenz[b,e]oxepine derivative or a salt thereof and a polyvalent weak acid, an acidic amino acid or an amide thereof, a basic amino acid or an amide thereof, or the salt thereof, and the like, for the purpose of providing an eye drop or a preparation for topical administration which enables the concentration of a dibenz [b,e]oxepin derivative or a salt thereof to be high; or an eye drop or a preparation for topical administration comprising the dibenz[b,e]oxepine derivative or the salt thereof and having an effective composition for treating allergic disturbance or inflammatory disorder of an eye or for treating allergic disturbance or inflammatory disorder of a nose.


Fig. 2

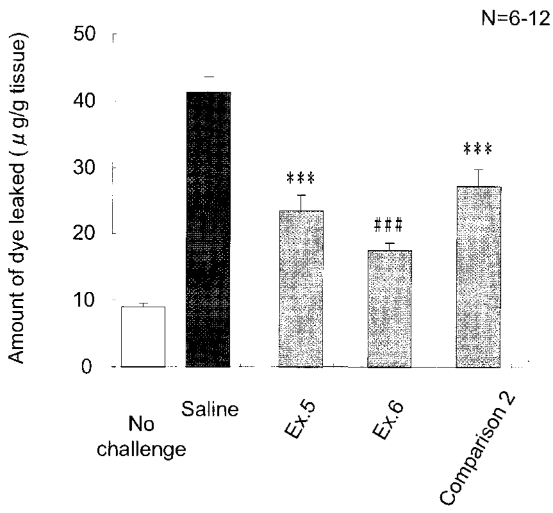


EP 2 189 157 A1

## Description

Technical Field

The present invention relates to an eye drop or a preparation for topical administration containing a dibenz [b, e] oxepin derivative or a salt thereof, and the like.

Background Art

It is known that a dibenz[b,e]oxepin derivative represented by the formula (I):
[Chemical 1]

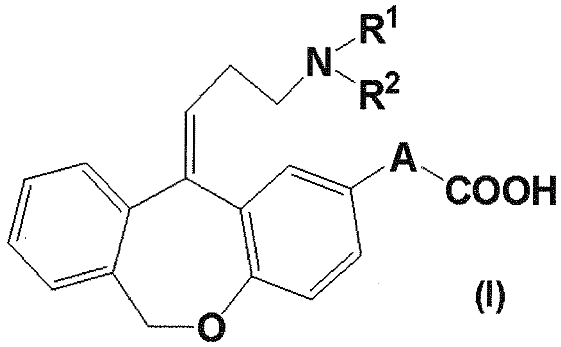

(I)

[wherein A represents a single bond, -CH=CH-, or $(CH_2)_n$-(wherein n represents an integer of 1 to 3); and $R^1$ and $R^2$ may be the same or different, and each represents hydrogen or lower alkyl, or are combined together with the adjacent nitrogen atom thereto to form a heterocyclic group] has an antiallergic activity and an antiinflammatory activity and is useful for allergic diseases such as nasal allergy and asthma and skin diseases such as urticaria (see Patent document 1). Further, an eye drop for eye diseases containing olopatadine hydrochloride which is one of the salts of dibenz[b,e] oxepin derivatives is known (see Patent documents 2 and 3).
However, olopatadine hydrochloride has a low solubility, and it is not easy to prepare a water-soluble eye drop in which the concentration of olopatadine is made high. In Patent document 3, in order to prepare an eye drop containing a high concentration of olopatadine, a solution composition which can be topically administered for treating allergic disturbance or inflammatory disorder of the eye and nose and contains a polymeric physical stability enhancing ingredient consisting essentially of polyvinylpyrrolidone or polystyrene sulfonic acid is proposed.
Patent document 1: JP-A-63-10784 (US-A-5116863)
Patent document 2: JP-A-09-510235 (US-A-5641805)
Patent document 3: JP-A-2004-536096 (US-B-6995186)

Disclosure of the Invention

Problems that the Invention is to Solve

An object of the present invention is to provide an eye drop or a preparation for topical administration having a composition which enables the concentration of a dibenz[b,e]oxepin derivative or a salt thereof to be high. Further, another object of the present invention is to provide an eye drop or a preparation for topical administration containing a dibenz[b,e]oxepin derivative or a salt thereof and having an effective composition for treating allergic disturbance or inflammatory disorder of the eye or for treating allergic disturbance or inflammatory disorder of a nose. Still another object of the present invention is to provide a medicament for allergic conjunctivitis or vernal conjunctivitis, which is an eye drop or a preparation for topical administration.

Means for Solving the Problems

The present invention relates to the following (1) to (36) :

(1) An eye drop or a preparation for topical administration, comprising; a dibenz[b,e]oxepin derivative represented by the formula (I):

[Chemical 2]

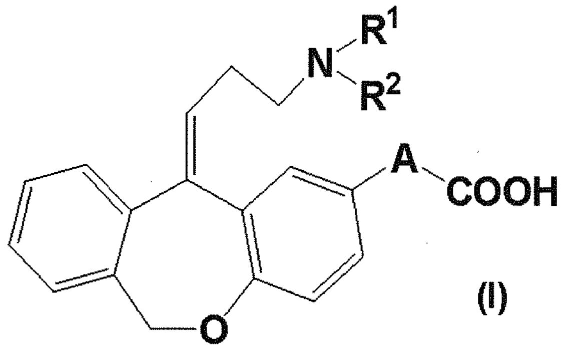

(I)

[wherein A represents a single bond, -CH=CH-, or $(CH_2)_{n-}$ (wherein n represents an integer of 1 to 3); and $R^1$ and $R^2$ may be the same or different, and each represents hydrogen or lower alkyl, or are combined together with the adjacent nitrogen atom thereto to form a heterocyclic group] or a salt thereof, and a polyvalent weak acid, an acidic amino acid or an amide thereof, a basic amino acid or an amide thereof, or a salt thereof;
(2) the eye drop or the preparation for topical administration according to the above (1), which further comprises a osmotic pressure adjusting agent;
(3) the eye drop or the preparation for topical administration according to the above (2), wherein the osmotic pressure adjusting agent is sodium chloride;
(4) the eye drop or the preparation for topical administration according to any one of the above (1) to (3), which further comprises a viscosity-enhancing agent;
(5) the eye drop or the preparation for topical administration according to the above (4), wherein the viscosity-enhancing agent is polyvinyl alcohol or polyvinylpyrrolidone;
(6) the eye drop or the preparation for topical administration according to the above (4) or (5), which has a pH of between 3.0 and 8.0;
(7) the eye drop or the preparation for topical administration according to any one of the above (1) to (5), which has a pH of between 3.0 and 5.5;
(8) the eye drop or the preparation for topical administration according to any one of the above (1) to (7), wherein the polyvalent weak acid, acidic amino acid or an amide thereof, basic amino acid or an amide thereof, or a salt thereof is phosphoric acid, boric acid, an acidic amino acid or an amide thereof, or a salt thereof;
(9) the eye drop or the preparation for topical administration according to any one of the above (1) to (7), wherein the polyvalent weak acid, acidic amino acid or an amide thereof, basic amino acid or an amide thereof, or a salt thereof is aspartic acid or an amide thereof, or a salt thereof;

(10) a medicament for allergic conjunctivitis or vernal conjunctivitis, which is an eye drop or a preparation for topical administration comprising a dibenz[b,e]oxepin derivative represented by the formula (I):

[Chemical 3]

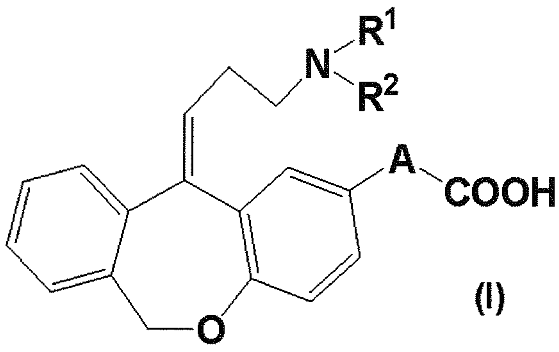

(I)

[wherein A represents a single bond, -CH=CH-, or $(CH_2)_n$-(wherein n represents an integer of 1 to 3) ; and $R^1$ and $R^2$ may be the same or different, and each represents hydrogen or lower alkyl, or are combined together with the adjacent nitrogen atom thereto to form a heterocyclic group] or a salt thereof, and a polyvalent weak acid, an acidic amino acid or an amide thereof, a basic amino acid or an amide thereof, or a salt thereof;

(11) the medicament for allergic conjunctivitis or vernal conjunctivitis according to the above (10), which further comprises an osmotic pressure adjusting agent;
(12) the medicament for allergic conjunctivitis or vernal conjunctivitis according to the above (11), wherein the osmotic pressure adjusting agent is sodium chloride;
(13) the medicament for allergic conjunctivitis or vernal conjunctivitis according to any one of the above (10) to (12), which further comprises a viscosity-enhancing agent;
(14) the medicament for allergic conjunctivitis or vernal conjunctivitis according to the above (13), wherein the viscosity-enhancing agent is polyvinyl alcohol or polyvinylpyrrolidone;
(15) the medicament for allergic conjunctivitis or vernal conjunctivitis according to the above (13) or (14), which has a pH of between 3.0 and 8.0;
(16) the medicament for allergic conjunctivitis or vernal conjunctivitis according to any one of the above (10) to (14), which has a pH of between 3.0 and 5.5;
(17) the medicament for allergic conjunctivitis or vernal conjunctivitis according to any one of the above (10) to (16), wherein the polyvalent weak acid, acidic amino acid or an amide thereof, basic amino acid or an amide thereof, or a salt thereof is phosphoric acid, boric acid, an acidic amino acid or an amide thereof, or a salt thereof;
(18) the medicament for allergic conjunctivitis or vernal conjunctivitis according to any one of the above (10) to (16), wherein the polyvalent weak acid, acidic amino acid or an amide thereof, basic amino acid or an amide thereof, or a salt thereof is aspartic acid or an amide thereof, or a salt thereof;

(19) a method for treating allergic conjunctivitis or vernal conjunctivitis, including administering an eye drop or a preparation for topical administration comprising a dibenz [b,e] oxepin derivative represented by the formula (I) :

[Chemical 4]

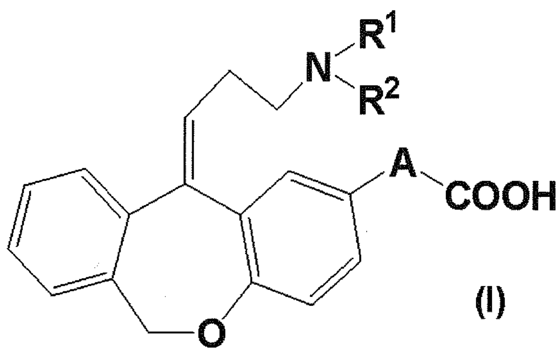

(I)

[wherein A represents a single bond, -CH=CH-, or $(CH_2)_n$-(wherein n represents an integer of 1 to 3); and $R^1$ and $R^2$ may be the same or different, and each represents hydrogen or lower alkyl, or are combined together with the adjacent nitrogen atom thereto to form a heterocyclic group] or a salt thereof, and a polyvalent weak acid, an acidic amino acid or an amide thereof, a basic amino acid or an amide thereof, or a salt thereof to a mammal;
(20) the method for treating allergic conjunctivitis or vernal conjunctivitis according to the above (19), wherein the eye drop or the preparation for topical administration further contains osmotic pressure adjusting agent;
(21) the method for treating allergic conjunctivitis or vernal conjunctivitis according to the above (20), wherein the osmotic pressure adjusting agent is sodium chloride;
(22) the method for treating allergic conjunctivitis or vernal conjunctivitis according to any one of the above (19) to (21), wherein the eye drop or the preparation for topical administration further contains viscosity-enhancing agent;
(23) the method for treating allergic conjunctivitis or vernal conjunctivitis according to the above (22), wherein the viscosity-enhancing agent is polyvinyl alcohol or polyvinylpyrrolidone;
(24) the method for treating allergic conjunctivitis or vernal conjunctivitis according to the above (22) or (23), wherein the eye drop or the preparation for topical administration has a pH of between 3.0 and 8.0;
(25) the method for treating allergic conjunctivitis or vernal conjunctivitis according to any one of the above (19) to (23), wherein the eye drop or the preparation for topical administration has a pH of between 3.0 and 5.5;
(26) the method for treating allergic conjunctivitis or vernal conjunctivitis according to any one of the above (19) to (25), wherein the polyvalent weak acid, acidic amino acid or an amide thereof, basic amino acid or an amide thereof, or a salt thereof is phosphoric acid, boric acid, an acidic amino acid or an amide thereof, or a salt thereof;
(27) the method for treating allergic conjunctivitis or vernal conjunctivitis according to any one of the above (19) to (25), wherein the polyvalent weak acid, acidic amino acid or an amide thereof, basic amino acid or an amide thereof, or a salt thereof is aspartic acid or an amide thereof, or a salt thereof;

(28) use of a dibenz[b,e]oxepin derivative represented by the formula (I):

[Chemical 5]

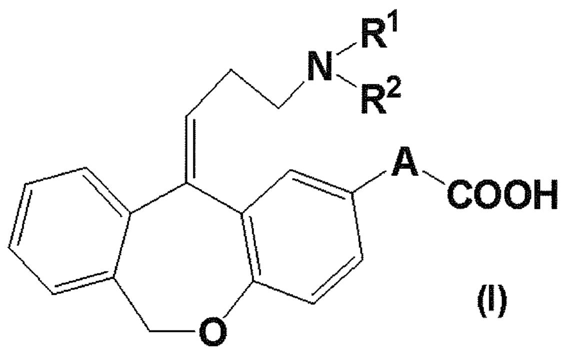

(I)

[wherein A represents a single bond, -CH=CH-, or $(CH_2)_n$-(wherein n represents an integer of 1 to 3); and $R^1$ and $R^2$ may be the same or different, and each represents hydrogen or lower alkyl, or are combined together with the adjacent nitrogen atom thereto to form a heterocyclic group] or a salt thereof, and a polyvalent weak acid, an acidic amino acid or an amide thereof, a basic amino acid or an amide thereof, or a salt thereof for the manufacture of a medicament for allergic conjunctivitis or vernal conjunctivitis in the form of an eye drop or a preparation for topical administration;
(29) the use according to the above (28), wherein the eye drop or the preparation for topical administration further contains osmotic pressure adjusting agent;
(30) the use according to the above (29), wherein the osmotic pressure adjusting agent is sodium chloride;
(31) the use according to any one of the above (28) to (30), wherein the eye drop or the preparation for topical administration further contains viscosity-enhancing agent; (32) the use according to the above (31), wherein the viscosity-enhancing agent is polyvinyl alcohol or polyvinylpyrrolidone;
(33) the use according to the above (31) or (32), wherein the eye drop or the preparation for topical administration has a pH of between 3.0 and 8.0;
(34) the use according to any one of the above (28) to (32), wherein the eye drop or the preparation for topical administration has a pH of between 3.0 and 5.5;
(35) the use according to any one of the above (28) to (34), wherein the polyvalent weak acid, acidic amino acid or an amide thereof, basic amino acid or an amide thereof, or a salt thereof is phosphoric acid, boric acid, an acidic amino acid or an amide thereof, or a salt thereof; and

(36) the use according to any one of the above (28) to (34), wherein the polyvalent weak acid, acidic amino acid or an amide thereof, basic amino acid or an amide thereof, or a salt thereof is aspartic acid or an amide thereof, or a salt thereof.

Effect of the Present invention

According to the present invention, an eye drop or a preparation for topical administration having a composition which enables the concentration of a dibenz[b,e]oxepin derivative or a salt thereof to be high can be provided. Further, an eye drop or a preparation for topical administration comprising a dibenz[b,e]oxepin derivative or a salt thereof and having an effective composition for treating allergic disturbance or inflammatory disorder of an eye or for treating allergic disturbance or inflammatory disorder of a nose can be provided. Further, a medicament for allergic conjunctivitis or vernal conjunctivitis, which is an eye drop or a preparation for topical administration can be provided.

Brief Description of the Drawings

[Fig. 1] Fig. 1 shows the results of a test for confirming that the preparations obtained in Examples 1 to 4 and Comparative Example 1 is effective in the treatment of allergic disturbance or inflammatory disorder of the eye (Test Example 3). The longitudinal axis indicates the amount of a dye per tissue weight; Ex. 1 to Ex. 4 indicate the cases where the preparations obtained in Examples 1 to 4 were administered, respectively; Comparison 1 indicates the case where the preparation obtained in Comparative Example 1 was administered; Saline indicates the case where physiological saline was administered; and No challenge indicates the untreated case. The single asterisk (*) indicates that there is a significant difference at a P value of less than 5% compared with the case where physiological saline was administered in a statistical analysis using Student's t-test. The double asterisk (**) indicates that there is a significant difference at a P value of less than 1% compared with the case where physiological saline was administered in a statistical analysis using Student's t-test.

[Fig. 2] Fig. 2 shows the results of a test for confirming that the preparations obtained in Examples 5 and 6 and Comparative Example 2 are effective in the treatment of allergic disturbance or inflammatory disorder of the eye (Test Example 4). The longitudinal axis indicates the amount of a dye per tissue weight; Ex. 5 and Ex. 6 indicate the cases where the preparations obtained in Examples 5 and 6 were administered, respectively; Comparison 2 indicates the case where the preparation obtained in Comparative Example 2 was administered; Saline indicates the case where physiological saline was administered; and No challenge indicates the untreated case. The triple asterisk (***) indicates that there is a significant difference at a P value of less than 0.1% compared with the case where physiological saline was administered in a statistical analysis using Student's t-test. The triple sharp (###) indicates that there is a significant difference at a P value of less than 0.1% compared with the case where physiological saline was administered in a statistical analysis using Aspin-Welch test.

[Fig. 3] Fig. 3 shows the results of a test for confirming that the preparations obtained in Examples 1 and 7 to 11 are effective in the treatment of allergic disturbance or inflammatory disorder of the eye (Test Example 5). The longitudinal axis indicates the amount of a dye per tissue weight; Ex. 1 and Ex. 7 to Ex. 11 indicate the cases where the preparations obtained in Examples 1 and 7 to 11 were administered, respectively; Saline indicates the case where physiological saline was administered; and No challenge indicates the untreated case. The triple asterisk (***) indicates that there is a significant difference at a P value of less than 0.1% compared with the case where physiological saline was administered in a statistical analysis using Student's t-test. The triple sharp (###) indicates that there is a significant difference at a P value of less than 0.1% compared with the case where physiological saline was administered in a statistical analysis using Aspin-Welch test.

[Fig. 4] Fig. 4 shows the results of a test for confirming that the preparations obtained in Examples 12 and 13 are effective in the treatment of allergic disturbance or inflammatory disorder of the eye (Test Example 5). The longitudinal axis indicates the amount of a dye per tissue weight; Ex. 12 and Ex. 13 indicate the cases where the preparations obtained in Examples 12 and 13 were administered, respectively; Saline indicates the case where physiological saline was administered; and No challenge indicates the untreated case. The triple asterisk (***) indicates that there is a significant difference at a P value of less than 0.1% compared with the case where physiological saline was administered in a statistical analysis using Student's t-test.

Best Mode for Carrying Out the Present invention

The eye drop or the preparation for topical administration of the present invention are an eye drop or a preparation for topical administration each comprising a dibenz[b,e]oxepin derivative or a salt thereof as an active ingredient, and further comprising a polyvalent weak acid, an acidic amino acid or an amide thereof, a basic amino acid or an amide thereof, or a salt thereof. The eye drop according to the present invention is a solution or a suspension of a dibenz [b,

e] oxepin derivative or a salt thereof, or is used by dissolving or suspending a dibenz [b, e] oxepin derivative or a salt thereof just before use, and is preferably an aqueous solution of a dibenz [b,e]oxepin derivative or a salt thereof. The preparation for topical administration according to the present invention is an eye ointment or an ointment preparation (including a cream) of a dibenz [b, e] oxepin derivative or a salt thereof, a liquid preparation thereof to be administered to the nose, lung, or skin, or a powder preparation thereof for powder inhalation, and is preferably an aqueous eye ointment or an aqueous ointment preparation of a dibenz[b,e]oxepin derivative or a salt thereof, or an aqueous liquid preparation thereof to be administered to the nose, lung, or skin.

The dibenz[b,e]oxepin derivative according to the present invention is a dibenz[b,e]oxepin derivative (hereinafter referred to as Compound (I)) represented by the formula (I).

[Chemical 6]

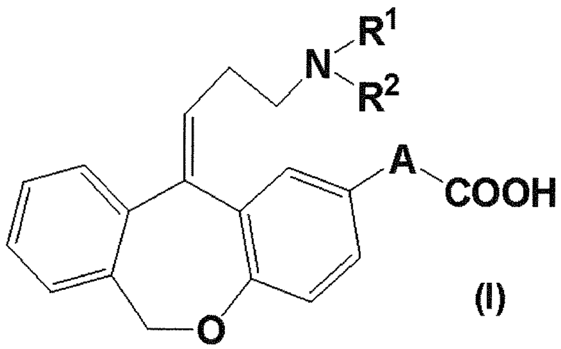

(I)

[In the formula, A represents a single bond, -CH=CH-, or $(CH_2)_n$-(wherein n represents an integer of 1 to 3); and $R^1$ and $R^2$ may be the same or different, and each represents hydrogen or lower alkyl, or are combined together with the adjacent nitrogen atom thereto to form a heterocyclic group.]

The definitions of the respective groups in the formula (I) are as follows. Examples of the lower alkyl include linear or branched alkyl each having 1 to 6 carbon atoms. Specific examples thereof include methyl, ethyl, propyl, isopropyl, butyl, isobutyl, sec-butyl, tert-butyl, pentyl, hexyl, and the like. Examples of the heterocyclic group to be formed together with the adjacent nitrogen atom include pyrrolidinyl, morpholino, thiomorpholino, N-methylpiperadinyl, pyrazolidinyl, piperidino, piperadinyl, indolyl, isoindolyl, and the like.

Examples of the salt of a dibenz[b,e]oxepin derivative include pharmaceutically acceptable acid addition salts, metal salts, ammonium salts, organic amine addition salts, amino acid addition salts, and the like. Examples of the pharmaceutically acceptable acid addition salts include inorganic acid salts such as hydrochloride, hydrobromide, nitrate, sulfate, and phosphate; and organic acid salts such as acetate, maleate, fumarate, tartrate, and citrate. Examples of the pharmaceutically acceptable metal salts include alkali metal salts such as lithium salts, sodium salts, and potassium salts; alkaline earth metal salts such as magnesium salts and calcium salts; aluminum salts, zinc salts, and the like. Examples of the pharmaceutically acceptable ammonium salts include salts of ammonium, tetramethylammonium, and the like. Examples of the pharmaceutically acceptable organic amine addition salts include addition salts of morpholine, piperidine, and the like. Examples of the pharmaceutically acceptable amino acid addition salts include addition salts of lysine, glycine, phenylalanine, aspartic acid, glutamic acid, and the like.

Compound (I) can be produced by or in accordance with by a method disclosed in Japanese Published Unexamined Patent Application No. 63-10784 or a modified method thereof. Among Compounds (I), a compound in which A is $CH_2$, and $R^1$ and $R^2$ are both $CH_3$ is preferred. Preferred specific examples of Compound (I) and a pharmaceutically acceptable salt thereof include (Z)-11-(3-dimethylaminopropylidene)-6,11-dihydrodibenz[b,e ]oxepin-2-acetic acid hydrochloride (hereinafter referred to as Compound (A)) represented by the formula (A).

[Chemical 7]

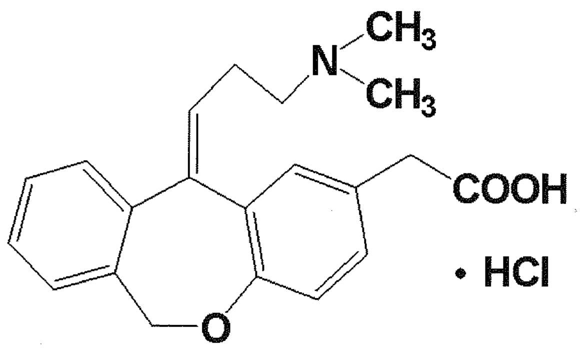

(A)

The eye drop or the preparation for topical administration of the present invention each contain a dibenz[b,e] oxepin derivative or a salt thereof in an amount of, preferably from 0.17 to 0.65% (hereinafter, all the amounts of components are expressed in terms of % (w/v)), more preferably from 0.17 to 0.35%, further more preferably from 0.18 to 0.32%.

Examples of the polyvalent weak acid in the present invention include phosphoric acid, boric acid, organic acids (such as citric acid and maleic acid), and the like, and preferred examples thereof include phosphoric acid, boric acid, and the like.

Examples of thecidic amino acid in the present invention include glutamic acid, aspartic acid, and the like. Further, examples of themide of an acidic amino acid include an acid amide formed from one or two carboxylic acids of an acidic amino acid and ammonia or ethylamine, an acid amide formed from an amino group at the α-position of an acidic amino acid and acetic acid or a fatty acid having 3 to 20 carbon atoms, and the like, and preferred examples thereof include glutamine, asparagine, γ-glutamylethylamide (theanine), γ-asparagylethylamide, and the like.

Examples of the basic amino acid in the present invention include lysine, arginine, histidine, ornithine, and the like.

Examples of the salt of a polyvalent weak acid, an acidic amino acid or an amide thereof, or a basic amino acid or an amide thereof include pharmaceutically acceptable acid addition salts, metal salts, ammonium salts, organic amine addition salts, amino acid addition salts, and the like. Examples of the pharmaceutically acceptable acid addition salts include inorganic acid salts such as hydrochloride, hydrobromide, nitrate, sulfate, and phosphate; and organic acid salts such as acetate, maleate, fumarate, tartrate, and citrate. Examples of the pharmaceutically acceptable metal salts include alkali metal salts such as lithium salts, sodium salts, and potassium salts; alkaline earth metal salts such as magnesium salts and calcium salts; aluminum salts, zinc salts, and the like. Examples of the pharmaceutically acceptable ammonium salts include salts of ammonium, tetramethylammonium, and the like. Examples of the pharmaceutically acceptable organic amine addition salts include addition salts of morpholine, piperidine, and the like. Examples of the pharmaceutically acceptable amino acid addition salts include addition salts of lysine, glycine, phenylalanine, aspartic acid, glutamic acid, and the like. Preferred examples of the salt of a polyvalent weak acid, an acidic amino acid or an amide thereof, or a basic amino acid or an amide thereof include pharmaceutically acceptable metal salts, and specific examples thereof include alkali metal salts such as lithium salts, sodium salts, and potassium salts; alkaline earth metal salts such as magnesium salts and calcium salts; aluminum salts, zinc salts, and the like.

The polyvalent weak acid, acidic amino acid or an amide thereof, basic amino acid or an amide thereof, or a salt thereof in the present invention can be used alone or in combination of two or more kinds thereof, and the phrase "comprising a polyvalent weak acid, an acidic amino acid or an amide thereof, a basic amino acid or an amide thereof, or a salt thereof" as used herein means "comprising a polyvalent weak acid, an acidic amino acid or an amide thereof, a basic amino acid or an amide thereof, or a salt thereof in the end", and there is no limitation on the starting materials. For example, the phrase "comprising glutamic acid" encompasses any case in which glutamic acid, glutamic acid and sodium hydroxide, sodium glutamate and hydrochloric acid, etc. is/are dissolved in water as (a) starting material(s), and the phrase "comprising glutamic acid and sodium glutamate" encompasses any case in which glutamic acid and sodium glutamate, glutamic acid and sodium hydroxide, sodium glutamate and hydrochloric acid, etc. are dissolved in water as starting materials.

Further, in the case where a pharmaceutically acceptable acid addition salt of a polyvalent weak acid is used as the salt of a dibenz [b, e] oxepin derivative, and in the case where a pharmaceutically acceptable acid addition salt of an acidic amino acid or an amide thereof, or a basic amino acid or an amide thereof is used as the salt of a dibenz

[b,e]oxepin derivative, by the incorporation of one component thereof, the dibenz[b,e]oxepin derivative or a salt thereof, and a polyvalent weak acid, an acidic amino acid or an amide thereof, a basic amino acid or an amide thereof, or a salt thereof in the present invention are contained, that is, two components turn out to be contained, and the above-mentioned embodiment is also encompassed in the present invention.

The polyvalent weak acid, acidic amino acid or an amide thereof, basic amino acid or an amide thereof, or a salt thereof in the present invention is more preferably phosphoric acid, boric acid, an acidic amino acid or an amide thereof, or a salt thereof, further more preferably glutamic acid or an amide thereof, aspartic acid or an amide thereof, or a salt thereof, and the most preferably aspartic acid or an amide thereof, or a salt thereof.

As for the concentration of the polyvalent weak acid, acidic amino acid or an amide thereof, basic amino acid or an amide thereof, or a salt thereof in the present invention, the total concentration of the polyvalent weak acid, acidic amino acid or an amide thereof, basic amino acid or an amide thereof, and a salt thereof is preferably from 0.1 to 100 mmol/L, more preferably from 1 to 50 mmol/L, further more preferably from 10 to 25 mmol/L.

The eye drop or the preparation for topical administration of the present invention each preferably contain viscosity-enhancing agent. Examples of the viscosity-enhancing agent in the present invention include polyvinyl alcohol, polyvinylpyrrolidone, chondroitin sulfate, dextran, dextran sulfate, chitosan, gelatin, sucrose, methyl cellulose, metal salts of alginic acid, metal salts of hyaluronic acid, and the like, and more preferred examples thereof include polyvinyl alcohol, polyvinylpyrrolidone, chondroitin sulfate, dextran, dextran sulfate, methyl cellulose, metal salts of alginic acid, metal salts of hyaluronic acid, and the like. These compounds can be used alone or in combination of two or more kinds thereof. Further, incorporation of polyvinyl alcohol, chondroitin sulfate, dextran, dextran sulfate, chitosan, and the like, each of which is a polymer having a low peroxide value among the viscosity-enhancing agents is preferred in the eye drop or the preparation for topical administration of the present invention. In the case where the eye drop or the preparation for topical administration of the present invention each contain viscosity-enhancing agent, they contain viscosity-enhancing agent in an amount of preferably from 0.01 to 5.0%, more preferably from 0.1 to 3.0%, further more preferably from 0.1 to 2.0%.

The eye drop or the preparation for topical administration of the present invention each has a pH of preferably between 3.0 and 5.5, more preferably between 3.5 and 5.0, further more preferably between 3.5 and 4.5.

Further, in the case where viscosity-enhancing agent having a property of improving the solubility of a drug is used as the viscosity-enhancing agent of the present invention, a pH that does not cause deposition of a dibenz[b,e]oxepin derivative or a salt thereof can be considered to be a preferred embodiment of the present invention. The pH in this case is preferably between 3.0 and 8.5, more preferably between 5.5 and 8.0, further more preferably between 6.5 and 7.5, and examples of the viscosity-enhancing agent in this case include polyvinyl alcohol, polyvinylpyrrolidone, and the like.

Further, examples of a more preferred combination of the polyvalent weak acid, acidic amino acid or an amide thereof, basic amino acid or an amide thereof, or a salt thereof with the viscosity-enhancing agent in the present invention include a combination of one or more members selected from acidic amino acids or amides thereof, basic amino acids or amides thereof, and salts thereof with one or more members selected from polyvinyl alcohol, polyvinylpyrrolidone, chondroitin sulfate, dextran, dextran sulfate, methyl cellulose, metal salts of alginic acid, and metal salts of hyaluronic acid, and examples of a more preferred combination include a combination of one or more members selected from acidic amino acids or amides thereof and salts thereof with polyvinyl alcohol.

The eye drop or the preparation for topical administration of the present invention may each contain an additive as needed in addition to the viscosity-enhancing agent. As the additive, a pH adjusting agent, a preservative, osmotic pressure adjusting agent or the like which is commonly used in an eye drop or a preparation for topical administration can be used. However, the eye drop or the preparation for topical administration of the present invention each preferably contain osmotic pressure adjusting agent in a sufficient amount so as to have an osmotic pressure (generally from 150 to 450 mOsm, preferably from 250 to 350 mOsm) which enables uptake thereof by, preferably, the eye, nasal mucosa, or the like. Examples of the osmotic pressure adjusting agent include sodium chloride, glycerin, boric acid, and the like, and preferred examples thereof include sodium chloride. Examples of the pH adjusting agent include hydrochloric acid, sodium hydroxide, and the like. Examples of the preservative include benzalkonium chloride, benzethonium chloride, p-hydroxybenzoic acid, methyl p-hydroxybenzoate, ethyl p-hydroxybenzoate, propyl p-hydroxybenzoate, butyl p-hydroxybenzoate, chlorobutanol, sorbic acid, edetic acid, or pharmaceutically acceptable salts thereof, and the like, and these compounds can be used alone or in combination of two or more kinds thereof.

The eye drop or the preparation for topical administration of the present invention can be used for treating allergic disturbance or inflammatory disorder of the eye, for example, for the treatment of allergic conjunctivitis or vernal conjunctivitis. Examples of the treatment of allergic conjunctivitis or vernal conjunctivitis include reduction in inflammation, suppression of inflammatory pain, itching, hyperemia, tear flow or gum, etc., and the like.

Further, the preparation of topical administration of the present invention can be used for treating allergic disturbance or inflammatory disorder of the nose, for example, for the treatment of allergic rhinitis, spring allergic rhinitis, or chronic allergic rhinitis. Examples of the treatment of allergic rhinitis, spring allergic rhinitis, or chronic allergic rhinitis include reduction in inflammation, suppression of inflammatory pain, itching, sneezing, nasal congestion, runny nose,

etc., and the like.

That is, the present invention also provides a method for treating allergic conjunctivitis or vernal conjunctivitis, including administering the eye drop or the preparation for topical administration of the present invention described above to a mammal, and the like. A target to which the eye drop or the preparation for topical administration is administered is preferably a human.

The medicament for allergic conjunctivitis or vernal conjunctivitis of the present invention is a medicament for allergic conjunctivitis or vernal conjunctivitis which is an eye drop or a preparation for topical administration of the present invention, and is preferably in the dosage form of an eye drop.

Further, the present invention also provides use of the dibenz [b, e] oxepin derivative or a salt thereof, and polyvalent weak acid, acidic amino acid or an amide thereof, basic amino acid or an amide thereof, or a salt thereof described above for the manufacture of a medicament for allergic conjunctivitis or vernal conjunctivitis in the form of an eye drop or a preparation for topical administration.

According to the present invention, an eye drop or a preparation for topical administration comprising a dibenz [b,e]oxepin derivative or a salt thereof which can be produced by or in accordance with by a simple preparation method and in which the chemical stability of the dibenz[b,e]oxepin derivative or a salt thereof is excellent, and a medicament for allergic conjunctivitis or vernal conjunctivitis which is an eye drop or a preparation for topical administration can also be provided.

Further, an eye drop or a preparation for topical administration containing a dibenz[b,e]oxepin derivative or a salt thereof in which the sustainability of an efficacy is promoted, and a medicament for allergic conjunctivitis or vernal conjunctivitis which is an eye drop or a preparation for topical administration can also be provided.

Hereinafter, the present invention is described in more detail with reference to Test Examples, however, the present invention is not limited to these Test Examples.

Test Example 1

100 mg of olopatadine hydrochloride (Kyowa Hakko Kogyo Co., Ltd., the same applies hereinafter) was weighed, and 10 mL of a glutamic acid-sodium glutamate buffer at pH 4.0 or pH 7.0 prepared by adding an aqueous solution of sodium hydroxide (Wako Pure Chemical Industries, Ltd. or Kanto Chemical Co., Inc. , the same applies hereinafter) to 0. 02 mol/L of L-glutamic acid (Wako Pure Chemical Industries, Ltd., the same applies hereinafter) was added thereto. Each of the resulting mixtures was stirred with a magnetic stirrer at room temperature (about 23°C) for 1 hour, and external observation and pH measurement were carried out. Then, an aqueous solution of sodium hydroxide was added to each mixture to prepare mixtures of pH 4 and 7, and external observation and pH measurement were carried out. Then, the mixtures were left at room temperature (about 23°C) for 43 hours, and external observation, pH measurement, solubility measurement, and osmotic pressure measurement were carried out. The results are shown in Table 1.

Method for solubility measurement

A test sample was diluted to 400-fold, and the concentration of olopatadine was measured by HPLC. In the case where precipitates were generated in the test sample, the test sample was filtered through a membrane filter (Ekicrodisc 25 (Pall Corporation)), and the resulting filtrate was diluted to 400-fold, and thereafter, the concentration of olopatadine was measured by HPLC.

Conditions for solubility measurement

HPLC: LC-10ADvp series · CLASS-VP Ver. 6.14 SP2 work station (Shimadzu Corporation)
Column: Inertsil C8, 4.6 mm (inner diameter) × 250 mm (length), particle diameter: 5 μm (GL Sciences, Inc.)
Detector: UV detector at 299 nm
Flow rate: 1.0 mL/min (adjusted such that the retention time of olopatadine becomes about 10 minutes)
Injection amount: 20 μL
Column temperature: 40°C (using a column oven)
Mobile phase: 2.3 g of sodium lauryl sulfate is added to a mixed solution of 0.05 mol/L of phosphate buffer solution (pH 3.5) and acetonitrile (55:45) and dissolved therein, and the final volume is made up to 1000 mL.
Rinse solution: water/acetonitrile (55:45)

Method for osmotic pressure measurement

An osmotic pressure was measured using an osmometer (Osmostat OM6040, Arkray, Inc.). Further, a ratio to the osmotic pressure of physiological saline was calculated according to the following equation.

$$(\text{osmotic pressure ratio}) = (\text{osmotic pressure of test liquid}) / (\text{osmotic pressure of physiological saline})\ (293\ \text{mOsm})$$

[Table 1]

| Liquid for dissolution | Glutamic acid-sodium glutamate buffer at pH 4.0 | | Glutamic acid-sodium glutamate buffer at pH 7.0 | |
|---|---|---|---|---|
| | Prepared to pH 4.0 | Prepared to pH 7.0 | Prepared to pH 4.0 | Prepared to pH 7.0 |
| External appearance after 1 hour stirring | Clear | Clear | Clear | Clear |
| pH after 1 hour stirring | 3.3 | 3.3 | 4.0 | 4.0 |
| pH adjustment (actual measurement value) | 4.1 | 6.9 | 4.0 | 6.9 |
| External appearance after pH adjustment | Clear | Turbid in white | Clear | Turbid in white |
| External appearance after 43 hours | Turbid in white | Turbid in white | Slightly turbid in white | Turbid in white |
| pH after 43 hours | 3.9 | 6.9 | 3.7 | 6.9 |
| Solubility after 43 hours (mg/mL) | 5.57 | 2.04 | 7.12 | 2.16 |
| Osmotic pressure ratio | 0.290 | 0.403 | 0.270 | 0.379 |

From Table 1, the glutamic acid-sodium glutamate buffers at pH 4.0 and pH 7.0 could dissolve 100 mg of olopatadine hydrochloride in 10 mL thereof, and the pH values of the respective solutions after dissolving olopatadine hydrochloride were 3.3 and 4.0, respectively. It was revealed that after a lapse of 43 hours from adjusting each liquid to pH 4 and 7, the solubility of olopatadine hydrochloride was higher in the liquid adjusted to pH 4 than the liquid adjusted to pH 7.

From the above results, it was revealed that by incorporating a polyvalent weak acid, an acidic amino acid or an amide thereof, a basic amino acid or an amide thereof, or a salt thereof and adjusting the pH to between 3.0 and 5.5, an eye drop or a preparation for topical administration containing a dibenz[b,e]oxepin derivative or a salt thereof at a high concentration can be prepared.

Test Example 2

100 mg of olopatadine hydrochloride was weighed, and 10 mL of a glutamic acid-sodium glutamate buffer at pH 4.0 or pH 7.0 prepared by adding an aqueous solution of sodium hydroxide to 0.02 mol/L of L-glutamic acid containing 0.9% sodium chloride (Wako Pure Chemical Industries, Ltd. , the same applies hereinafter) or 10 mL of an aqueous

solution of 0.9% sodium chloride (physiological saline) was added thereto. Each of the resulting mixtures was stirred with a magnetic stirrer at room temperature (about 23°C) for 1 hour, and external observation and pH measurement were carried out. Then, an aqueous solution of sodium hydroxide was added to each mixture to prepare a mixture of pH 4, and external observation and pH measurement were carried out. Then, the mixture was left at room temperature (about 23°C) for 43 hours, and external observation, pH measurement, solubility measurement, and osmotic pressure measurement were carried out in the same manner as in Test Example 1. The results are shown in Table 2.

[Table 2]

| Liquid for dissolution | Buffer at pH 4.0 containing 0.9% NaCl | Buffer at pH 7.0 containing 0.9% NaCl | Aqueous solution of 0.9% NaCl |
|---|---|---|---|
| External appearance after 1 hour stirring | Turbid in white | Almost clear | Turbid in white |
| pH after 1 hour stirring | 3.6 | 4.1 | 3.0 |
| pH adjustment (actual measurement value) | 3.8 | 4.1 | 3.8 |
| External appearance after pH adjustment | Turbid in white | Almost clear | Turbid in white |
| External appearance after 43 hours | Turbid in white | Turbid in white | Turbid in white |
| pH after 43 hours | 3.8 | 3.9 | 3.7 |
| Solubility after 43 hours (mg/mL) | 6.24 | 6.38 | 5.07 |
| Osmotic pressure ratio | 1.167 | 1.201 | 1.085 |

From Table 2, as for the cases where 100 mg of olopatadine hydrochloride was added to 10 mL of the glutamic acid-sodium glutamate buffer at pH 4.0 containing 0.9% sodium chloride, the glutamic acid-sodium glutamate buffer at pH 7.0 containing 0.9% sodium chloride, and the aqueous solution of 0.9% sodium chloride, and thereafter, the respective liquids were subjected to pH adjustment to pH 4, it was revealed that the solubility of olopatadine hydrochloride was higher in the cases where the glutamic acid-sodium glutamate buffer at pH 4.0 containing 0.9% sodium chloride and the glutamic acid-sodium glutamate buffer at pH 7.0 containing 0.9% sodium chloride were used as a liquid for dissolution than in the case where the aqueous solution of 0.9% sodium chloride was used as a liquid for dissolution.

From the above results, it was revealed that even in the case of containing osmotic pressure adjusting agent, by incorporating a polyvalent weak acid, an acidic amino acid or an amide thereof, a basic amino acid or an amide thereof, or a salt thereof and adjusting the pH to between 3.0 and 5.5, an eye drop or a preparation for topical administration containing a dibenz[b,e]oxepin derivative or a salt thereof at a higher concentration can be prepared.

Hereinafter, the present invention is described in more detail with reference to Examples, however, the present invention is not limited to these Examples.

The eye drop or the preparation for topical administration of the present invention can each be prepared by dissolving a polyvalent weak acid, an acidic amino acid or an amide thereof, a basic amino acid or an amide thereof, or a salt thereof and if necessary, an additive such as osmotic pressure adjusting agent in water, and thereafter adding a dibenz[b,e]oxepin derivative or a salt thereof thereto, and further adding a pH adjusting agent thereto to prepare a mixture with a pH of between 3.0 and 5.5, preferably between 3.5 and 5.0, more preferably between 3.5 and 4.5, or by dissolving a polyvalent weak acid, an acidic amino acid or an amide thereof, a basic amino acid or an amide thereof, or a salt thereof and if necessary, an additive such as osmotic pressure adjusting agent in water, and thereafter adding a dibenz [b,e]oxepin derivative or a salt thereof thereto, and further adding viscosity-enhancing agent and a pH adjusting agent thereto to prepare a mixture with a pH of between 3.0 and 8.5, and then, further adding water and if necessary an additive, or an additive and water or the like. More specifically, an eye drop or a preparation for topical administration can be prepared as described in the following Examples 1 to 13.

Example 1

74 mg of L-glutamic acid and 175 mg of sodium chloride were dissolved in 18 mL of water, and 50 mg of

olopatadine hydrochloride was added thereto. Then, an aqueous solution of sodium hydroxide and hydrochloric acid (Wako Pure Chemical Industries, Ltd. or Nacalai Tesque, Inc., the same applies hereinafter) were added thereto to prepare a mixture of pH 4. Then, water was further added thereto to make the total volume up to 25 mL, whereby an eye drop containing 0.2% olopatadine hydrochloride was obtained. The thus obtained eye drop had a pH of 4.0 and an osmotic pressure of 291 mOsm.

Example 2

74 mg of L-glutamic acid and 175 mg of sodium chloride were dissolved in 18 mL of water, and 50 mg of olopatadine hydrochloride was added thereto. Then, an aqueous solution of sodium hydroxide and hydrochloric acid were added thereto to prepare a mixture of pH 5. Then, water was further added thereto to make the total volume up to 25 mL, whereby an eye drop containing 0.2% olopatadine hydrochloride was obtained. The thus obtained eye drop had a pH of 5.0 and an osmotic pressure of 305 mOsm.

Example 3

78 mg of sodium dihydrogen phosphate (Wako Pure Chemical Industries, Ltd. , the same applies hereinafter) and 175 mg of sodium chloride were dissolved in 18 mL of water, and 50 mg of olopatadine hydrochloride was added thereto. Then, an aqueous solution of sodium hydroxide and hydrochloric acid were added thereto to prepare a mixture of pH 4. Then, water was further added thereto to make the total volume up to 25 mL, whereby an eye drop containing 0.2% olopatadine hydrochloride was obtained. The thus obtained eye drop had a pH of 4.0 and an osmotic pressure of 325 mOsm.

Example 4

78 mg of sodium dihydrogen phosphate and 175 mg of sodium chloride were dissolved in 18 mL of water, and 50 mg of olopatadine hydrochloride was added thereto. Then, an aqueous solution of sodium hydroxide and hydrochloric acid were added thereto to prepare a mixture of pH 5. Then, water was further added thereto to make the total volume up to 25 mL, whereby an eye drop containing 0.2% olopatadine hydrochloride was obtained. The thus obtained eye drop had a pH of 5.0 and an osmotic pressure of 351 mOsm.

Comparative Example 1

225 mg of sodium chloride was dissolved in 18 mL of water, and 50 mg of olopatadine hydrochloride was added thereto. Then, an aqueous solution of sodium hydroxide and hydrochloric acid were added thereto to prepare a mixture of pH 4. Then, water was further added thereto to make the total volume up to 25 mL, whereby an eye drop containing 0.2% olopatadine hydrochloride was obtained. The thus obtained eye drop had a pH of 4.0 and an osmotic pressure of 346 mOsm.

Example 5

74 mg of L-glutamic acid and 175 mg of sodium chloride were dissolved in 18 mL of water, and 75 mg of olopatadine hydrochloride was added thereto. Then, an aqueous solution of sodium hydroxide and hydrochloric acid were added thereto to prepare a mixture of pH 4. Then, water was further added thereto to make the total volume up to 25 mL, whereby an eye drop containing 0.3% olopatadine hydrochloride was obtained. The thus obtained eye drop had a pH of 4.0 and an osmotic pressure of 326 mOsm.

Example 6

67 mg of L-aspartic acid (Wako Pure Chemical Industries, Ltd., the same applies hereinafter) and 175 mg of sodium chloride were dissolved in 18 mL of water, and 75 mg of olopatadine hydrochloride was added thereto. Then, an aqueous solution of sodium hydroxide and hydrochloric acid were added thereto to prepare a mixture of pH 4. Then, water was further added thereto to make the total volume up to 25 mL, whereby an eye drop containing 0.3% olopatadine hydrochloride was obtained. The thus obtained eye drop had a pH of 4.0 and an osmotic pressure of 329 mOsm.

Comparative Example 2

225 mg of sodium chloride was dissolved in 18 mL of water, and 75 mg of olopatadine hydrochloride was added

thereto. Then, an aqueous solution of sodium hydroxide and hydrochloric acid were added thereto to prepare a mixture of pH 4. Then, water was further added thereto to make the total volume up to 25 mL, whereby an eye drop containing 0.3% olopatadine hydrochloride was obtained. The thus obtained eye drop had a pH of 4.0 and an osmotic pressure of 340 mOsm.

Example 7

67 mg of L-aspartic acid and 175 mg of sodium chloride were dissolved in 18 mL of water, and 50 mg of olopatadine hydrochloride was added thereto. Then, an aqueous solution of sodium hydroxide and hydrochloric acid were added thereto to prepare a mixture of pH 4. Then, water was further added thereto to make the total volume up to 25 mL, whereby an eye drop containing 0.2% olopatadine hydrochloride was obtained. The thus obtained eye drop had a pH of 4.0, an osmotic pressure of 331 mOsm, and a viscosity of 0.89 mPa·s.

Example 8

294 mg of L-glutamic acid, 700 mg of sodium chloride, and 45 mL of an aqueous solution of 4% polyvinylpyrrolidone (prepared by dissolving 4.00 g of polyvinylpyrrolidone K-30 (Junsei Chemical Co., Ltd.) in 80 mL of water, and adding an aqueous solution of sodium hydroxide and hydrochloric acid thereto to prepare a solution of pH 11.5, and then, adding water thereto to make the total volume up to 100 mL, followed by a heat treatment in a water bath at 70 to 75°C for 30 minutes, the same applies hereinafter) were dissolved in 35 mL of water, and 200 mg of olopatadine hydrochloride was added thereto. Then, an aqueous solution of sodium hydroxide and hydrochloric acid were added thereto to adjust the pH to 4. Then, water was further added thereto to make the total volume up to 100 mL, whereby an eye drop containing 0.2% olopatadine hydrochloride was obtained. The thus obtained eye drop had a pH of 4.0, an osmotic pressure of 270 mOsm, and a viscosity of 0.90 mPa·s.

Example 9

266 mg of L-aspartic acid, 700 mg of sodium chloride, and 45 mL of an aqueous solution of 4% polyvinylpyrrolidone were dissolved in 35 mL of water, and 200 mg of olopatadine hydrochloride was added thereto. Then, an aqueous solution of sodium hydroxide and hydrochloric acid were added thereto to prepare a mixture of pH 4. Then, water was further added thereto to make the total volume up to 100 mL, whereby an eye drop containing 0.2% olopatadine hydrochloride was obtained. The thus obtained eye drop had a pH of 4.0, an osmotic pressure of 280 mOsm, and a viscosity of 0.90 mPa·s.

Example 10

294 mg of L-glutamic acid, 700 mg of sodium chloride, and 18 mL of an aqueous solution of 10% polyvinyl alcohol (prepared by adding 80 mL of water to 10.00 g of polyvinyl alcohol 500 (Junsei Chemical Co. , Ltd.), heating the resulting mixture in a water bath at 80 to 90°C for 30 minutes to achieve complete dissolution, followed by cooling the resulting solution to room temperature and adding water thereto to make the total volume up to 100 mL, the same applies hereinafter) were dissolved in 70 mL of water, and 200 mg of olopatadine hydrochloride was added thereto. Then, an aqueous solution of sodium hydroxide and hydrochloric acid were added thereto to prepare a mixture of pH 4. Then, water was further added thereto to make the total volume up to 100 mL, whereby an eye drop containing 0.2% olopatadine hydrochloride was obtained. The thus obtained eye drop had a pH of 4.0, an osmotic pressure of 363 mOsm, and a viscosity of 1.83 mPa·s.

Example 11

266 mg of L-aspartic acid, 700 mg of sodium chloride, and 18 mL of an aqueous solution of 10% polyvinyl alcohol were dissolved in 70 mL of water, and 200 mg of olopatadine hydrochloride was added thereto. Then, an aqueous solution of sodium hydroxide and hydrochloric acid were added thereto to prepare a mixture of pH 4. Then, water was further added thereto to make the total volume up to 100 mL, whereby an eye drop containing 0.2% olopatadine hydrochloride was obtained. The thus obtained eye drop had a pH of 4.0, an osmotic pressure of 360 mOsm, and a viscosity of 1.83 mPa·s.

Example 12

133 mg of L-aspartic acid, 250 mg of sodium chloride, and 10 mL of an aqueous solution of 10% polyvinyl

alcohol were dissolved in 30 mL of water, and 100 mg of olopatadine hydrochloride was added thereto. Then, an aqueous solution of sodium hydroxide was added thereto to prepare a mixture of pH 7. Then, water was further added thereto to make the total volume up to 50 mL, whereby an eye drop containing 0.2% olopatadine hydrochloride was obtained. The thus obtained eye drop had a pH of 7.1 and an osmotic pressure of 268 mOsm.

Example 13

133 mg of L-aspartic acid, 250 mg of sodium chloride, and 10 mL of an aqueous solution of 10% polyvinyl alcohol were dissolved in 30 mL of water, and 100 mg of olopatadine hydrochloride was added thereto. Then, hydrochloric acid was added thereto to prepare a mixture of pH 4. Then, water was further added thereto to make the total volume up to 50 mL, whereby an eye drop containing 0.2% olopatadine hydrochloride was obtained. The thus obtained eye drop had a pH of 4.1 and an osmotic pressure of 269 mOsm.

Test Example 3

By using a disease model (allergic conjunctivitis), it was confirmed that the preparations obtained in Examples 1 to 4 and Comparative Example 1 are effective in the treatment of allergic disturbance or inflammatory disorder of the eye according to the following method.

Wistar rats (at 5 weeks of age) were passively sensitized by subconjunctival injection of rat anti-ovalbumin (OVA) serum at 0.03 mL/site into the palpebral conjunctiva of the right eye. After 48 hours, 1.5 w/v% Evans blue (Sigma-Aldrich) physiological saline containing 2 mg of OVA (Sigma-Aldrich, St. Louis, MO, USA) as an antigen was intravenously administered in a proportion of 1.0 mL per 200 g of body weight to challenge the animals. 30 minutes thereafter, the animals were sacrificed by carbon dioxide gas, and the subconjunctival tissue of the palpebral conjunctiva of the right eye and the eyeballs were excised and the weights thereof were measured. 10 μL of each of the preparations obtained in Examples 1 to 4 and Comparative Example 1 was instilled in the right and left eyes 24 hours prior to challenge. The amount of a dye in a blue-stained area was quantitatively determined according to the method of Tamura et al. (Tamura T, Sato H, Miki I, Suzuki K, Ohmori K, and Karasawa A. Effects of orally administered olopatadine hydrochloride on the ocular allergic reaction in rats. Allergol Int. 2003; 52: 77-83). That is, blue-stained tissue was placed in a polypropylene tube, and formamide (Wako Pure Chemical Industries, Ltd. Osaka) was added thereto. Then, the tube was heated to 45°C using a constant temperature dryer (Yamato Scientific. Co. , Ltd. , Tokyo), and let stand overnight to extract the dye. As for the leaking dye, the absorbance at 625 nm was measured using a microplate spectrophotometer (Molecular devices, Sunnyvale, CA, USA), and the amount of a dye was quantitatively determined from a calibration curve. The amount of a dye was expressed per tissue weight. A statistical analysis was performed using a statistical analysis software Statistical Analysis System (SAS; Release 9.1.3, SAS Institute, Cary, NC, USA), and F-test was used for comparison between two groups, Student's t-test was used for equal variance, and Aspin-Welch test was used for unequal variance. It was determined that there was a significant difference when the P value was less than 5%.
The results are shown in Fig. 1.

From Fig. 1, all the preparations of Examples 1 to 4 and Comparative Example 1 significantly exhibited an efficacy as compared with the case where physiological saline was administered (the column indicated by Saline in Fig. 1). Further, it was revealed that the preparations containing glutamic acid or phosphoric acid (Examples 1 to 4) exhibited a higher efficacy than the preparation without containing either compound (Comparative Example 1).

From the above results, it was revealed that by incorporating a polyvalent weak acid, an acidic amino acid or an amide thereof, a basic amino acid or an amide thereof, or a salt thereof and adjusting the pH to between 3.0 and 5.5, an eye drop or a preparation for topical administration containing a dibenz[b,e]oxepin derivative or a salt thereof at a higher concentration is effective in the treatment of allergic disturbance or inflammatory disorder of the eye.

Test Example 4

It was confirmed that the preparations obtained in Examples 5 and 6 and Comparative Example 2 are effective in the treatment of allergic disturbance or inflammatory disorder of the eye in the same manner as in Test Example 3.
The results are shown in Fig. 2.

From Fig. 2, all the preparations of Examples 5 and 6 and Comparative Example 2 significantly exhibited an efficacy as compared with the case where physiological saline was administered (the column indicated by Saline in Fig. 2). Further, it was revealed that the preparations containing glutamic acid or aspartic acid (Examples 5 and 6) exhibited a higher efficacy than the preparation without containing either compound (Comparative Example 2). Further, it was revealed that the preparation containing aspartic acid (Example 6) exhibited a higher efficacy than the preparation containing glutamic acid (Example 5).

Test Example 5

By using a disease model (allergic conjunctivitis), it was confirmed that the preparations obtained in Examples 1 and 7 to 13 are effective in the treatment of allergic disturbance or inflammatory disorder of the eye according to the following method.

Wistar rats (at 5 weeks of age) were passively sensitized by subconjunctival injection of rat anti-ovalbumin (OVA) serum at 0.03 mL/site into the palpebral conjunctiva of the right eye. After 48 hours, 1.5 w/v% Evans blue (Sigma-Aldrich) physiological saline containing 2 mg of OVA (Sigma-Aldrich, St. Louis, MO, USA) as an antigen was intravenously administered in a proportion of 1.0 mL per 200 g of body weight to challenge the animals. 30 minutes thereafter, the animals were sacrificed by carbon dioxide gas, and the subconjunctival tissue of the palpebral conjunctiva of the right eye and the eyeballs were excised and the weights thereof were measured. 10 μL of each of the preparations obtained in Examples 1 and 7 to 13 was instilled in the right and left eyes 6 or 24 hours prior to challenge. The amount of a dye in a blue-stained area was quantitatively determined according to the method of Tamura et al. (Tamura T, SatoH, Miki I, Suzuki K, Ohmori K, and Karasawa A. Effects of orally administered olopatadine hydrochloride on the ocular allergic reaction in rats. Allergol Int. 2003; 52: 77-83). That is, blue-stained tissue was placed in a polypropylene tube, and formamide (Wako Pure Chemical Industries, Ltd. Osaka) was added thereto. Then, the tube was heated to 45°C using a constant temperature dryer (Yamato Scientific. Co., Ltd., Tokyo), and let stand overnight to extract the dye. As for the leaking dye, the absorbance at 625 nm was measured using a microplate spectrophotometer (Molecular devices, Sunnyvale, CA, USA), and the amount of a dye was quantitatively determined from a calibration curve. The amount of a dye was expressed per tissue weight. A statistical analysis was performed using a statistical analysis software Statistical Analysis System (SAS; Release 9. 1.3, SAS Institute, Cary, NC, USA), and F-test was used for comparison between two groups, Student's t-test was used for equal variance, and Aspin-Welch test was used for unequal variance. It was determined that there was a significant difference when the P value was less than 5%.
The results are shown in Fig. 3 and Fig. 4.

From Fig. 3, all the preparations of Examples 1 and 7 to 11 significantly exhibited an efficacy in both cases where OVA was administered as an antigen 6 and 24 hours after administering the preparation as compared with the case where physiological saline was administered (the column indicated by Saline in Fig. 3). Further, it was revealed that the preparations containing polyvinyl alcohol (Examples 10 and 11) exhibited a higher efficacy than the preparations without containing viscosity-enhancing agent (Examples 1 and 7) and the preparations containing polyvinylpyrrolidone (Examples 8 and 9).

Further, from Fig. 4, both preparations of Examples 12 and 13 significantly exhibited an efficacy in both cases where OVA was administered as an antigen 6 and 24 hours after administering the preparation as compared with the case where physiological saline was administered (the column indicated by Saline in Fig. 4).

From the above results, it was revealed that by incorporating a polyvalent weak acid, an acidic amino acid or an amide thereof, a basic amino acid or an amide thereof, or a salt thereof and adjusting the pH to between 3.0 and 5.5, an eye drop or a preparation for topical administration containing a dibenz[b,e]oxepin derivative or a salt thereof at a higher concentration, and by further incorporating viscosity-enhancing agent and adjusting the pH to between 3.0 and 8.0, an eye drop or a preparation for topical administration containing a dibenz[b,e]oxepin derivative or a salt thereof at a higher concentration are effective in the treatment of allergic disturbance or inflammatory disorder of the eye.

Industrial Applicability

According to the present invention, an eye drop or a preparation for topical administration having a composition which enables the concentration of a dibenz[b,e]oxepin derivative or a salt thereof to be high can be provided. Further, an eye drop or a preparation for topical administration containing a dibenz[b,e]oxepin derivative or a salt thereof and having an effective composition for treating allergic disturbance or inflammatory disorder of an eye or for treating allergic disturbance or inflammatory disorder of a nose can be provided. Further, a medicament for allergic conjunctivitis or vernal conjunctivitis, which is an eye drop or a preparation for topical administration can be provided.

## Claims

**1.** An eye drop or a preparation for topical administration, comprising; a dibenz [b, e] oxepin derivative represented by the formula (I):

[Chemical 8]

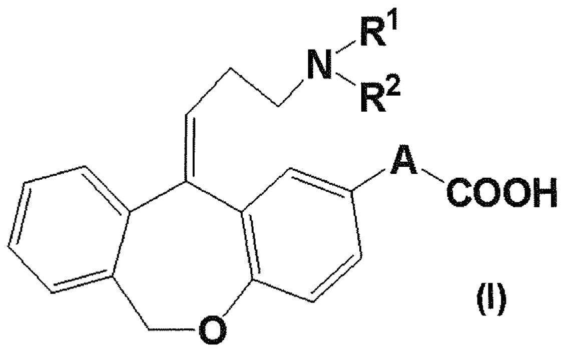

(I)

[wherein A represents a single bond, -CH=CH-, or $(CH_2)_n$-(wherein n represents an integer of 1 to 3); and $R^1$ and $R^2$ may be the same or different, and each represents hydrogen or lower alkyl, or are combined together with the adjacent nitrogen atom thereto to form a heterocyclic group] or a salt thereof, and a polyvalent weak acid, an acidic amino acid or an amide thereof, a basic amino acid or an amide thereof, or a salt thereof.

2. The eye drop or the preparation for topical administration according to Claim 1, which further comprises an osmotic pressure adjusting agent.

3. The eye drop or the preparation for topical administration according to Claim 2, wherein the osmotic pressure adjusting agent is sodium chloride.

4. The eye drop or the preparation for topical administration according to any one of Claims 1 to 3, which further comprises a viscosity-enhancing agent.

5. The eye drop or the preparation for topical administration according to Claim 4, wherein the viscosity-enhancing agent is polyvinyl alcohol or polyvinylpyrrolidone.

6. The eye drop or the preparation for topical administration according to Claim 4 or 5, which has a pH of between 3.0 and 8.0.

7. The eye drop or the preparation for topical administration according to any one of Claims 1 to 5, which has a pH of between 3.0 and 5.5.

8. The eye drop or the preparation for topical administration according to any one of Claims 1 to 7, wherein the polyvalent weak acid, acidic amino acid or an amide thereof, basic amino acid or an amide thereof, or a salt thereof is phosphoric acid, boric acid, an acidic amino acid or an amide thereof, or a salt thereof.

9. The eye drop or the preparation for topical administration according to any one of Claims 1 to 7, wherein the polyvalent weak acid, acidic amino acid or an amide thereof, basic amino acid or an amide thereof, or a salt thereof is aspartic acid or an amide thereof, or a salt thereof.

10. A medicament for allergic conjunctivitis or vernal conjunctivitis, which is an eye drop or a preparation for topical administration comprising a dibenz[b,e]oxepin derivative represented by the formula (I):

[Chemical 9]

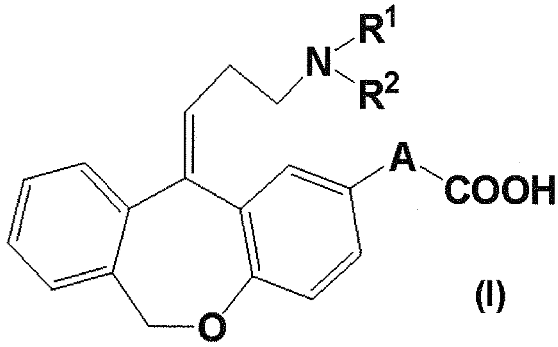

(I)

[wherein A represents a single bond, -CH=CH-, or $(CH_2)_n$-(wherein n represents an integer of 1 to 3); and $R^1$ and $R^2$ may be the same or different, and each represents hydrogen or lower alkyl, or are combined together with the adjacent nitrogen atom thereto to form a heterocyclic group] or a salt thereof, and a polyvalent weak acid, an acidic amino acid or an amide thereof, a basic amino acid or an amide thereof, or a salt thereof.

**11.** The medicament for allergic conjunctivitis or vernal conjunctivitis according to Claim 10, which further comprises an osmotic pressure adjusting agent.

**12.** The medicament for allergic conjunctivitis or vernal conjunctivitis according to Claim 11, wherein the osmotic pressure adjusting agent is sodium chloride.

**13.** The medicament for allergic conjunctivitis or vernal conjunctivitis according to any one of Claims 10 to 12, which further comprises a viscosity-enhancing agent.

**14.** The medicament for allergic conjunctivitis or vernal conjunctivitis according to Claim 13, wherein the viscosity-enhancing agent is polyvinyl alcohol or polyvinylpyrrolidone.

**15.** The medicament for allergic conjunctivitis or vernal conjunctivitis according to Claim 13 or 14, which has a pH of between 3.0 and 8.0.

**16.** The medicament for allergic conjunctivitis or vernal conjunctivitis according to any one of Claims 10 to 14, which has a pH of between 3.0 and 5.5.

**17.** The medicament for allergic conjunctivitis or vernal conjunctivitis according to any one of Claims 10 to 16, wherein the polyvalent weak acid, acidic amino acid or an amide thereof, basic amino acid or an amide thereof, or a salt thereof is phosphoric acid, boric acid, an acidic amino acid or an amide thereof, or a salt thereof.

**18.** The medicament for allergic conjunctivitis or vernal conjunctivitis according to any one of Claims 10 to 16, wherein the polyvalent weak acid, acidic amino acid or an amide thereof, basic amino acid or an amide thereof, or a salt thereof is aspartic acid or an amide thereof, or a salt thereof.

**19.** A method for treating allergic conjunctivitis or vernal conjunctivitis, comprising administering an eye drop or a preparation for topical administration comprising a dibenz [b,e] oxepin derivative represented by the formula (I):

[Chemical 10]

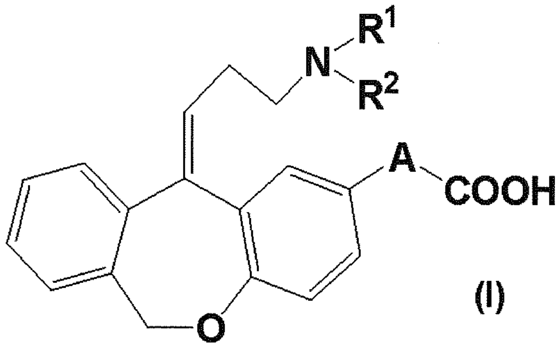

(I)

[wherein A represents a single bond, -CH=CH-, or $(CH_2)_n$-(wherein n represents an integer of 1 to 3) ; and $R^1$ and $R^2$ may be the same or different, and each represents hydrogen or lower alkyl, or are combined together with the adjacent nitrogen atom thereto to form a heterocyclic group] or a salt thereof, and a polyvalent weak acid, an acidic amino acid or an amide thereof, a basic amino acid or an amide thereof, or a salt thereof to a mammal.

**20.** Use of a dibenz[b,e]oxepin derivative represented by the formula (I):

[Chemical 11]

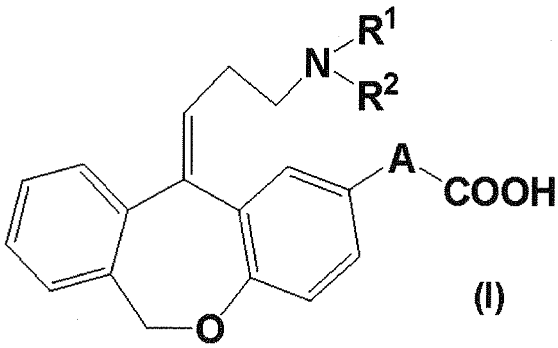

(I)

[wherein A represents a single bond, -CH=CH-, or $(CH_2)_n$-(wherein n represents an integer of 1 to 3); and $R^1$ and $R^2$ may be the same or different, and each represents hydrogen or lower alkyl, or are combined together with the adjacent nitrogen atom thereto to form a heterocyclic group] or a salt thereof, and a polyvalent weak acid, an acidic amino acid or an amide thereof, a basic amino acid or an amide thereof, or a salt thereof for the manufacture of a medicament for allergic conjunctivitis or vernal conjunctivitis in the form of an eye drop or a preparation for topical administration.

Fig. 1

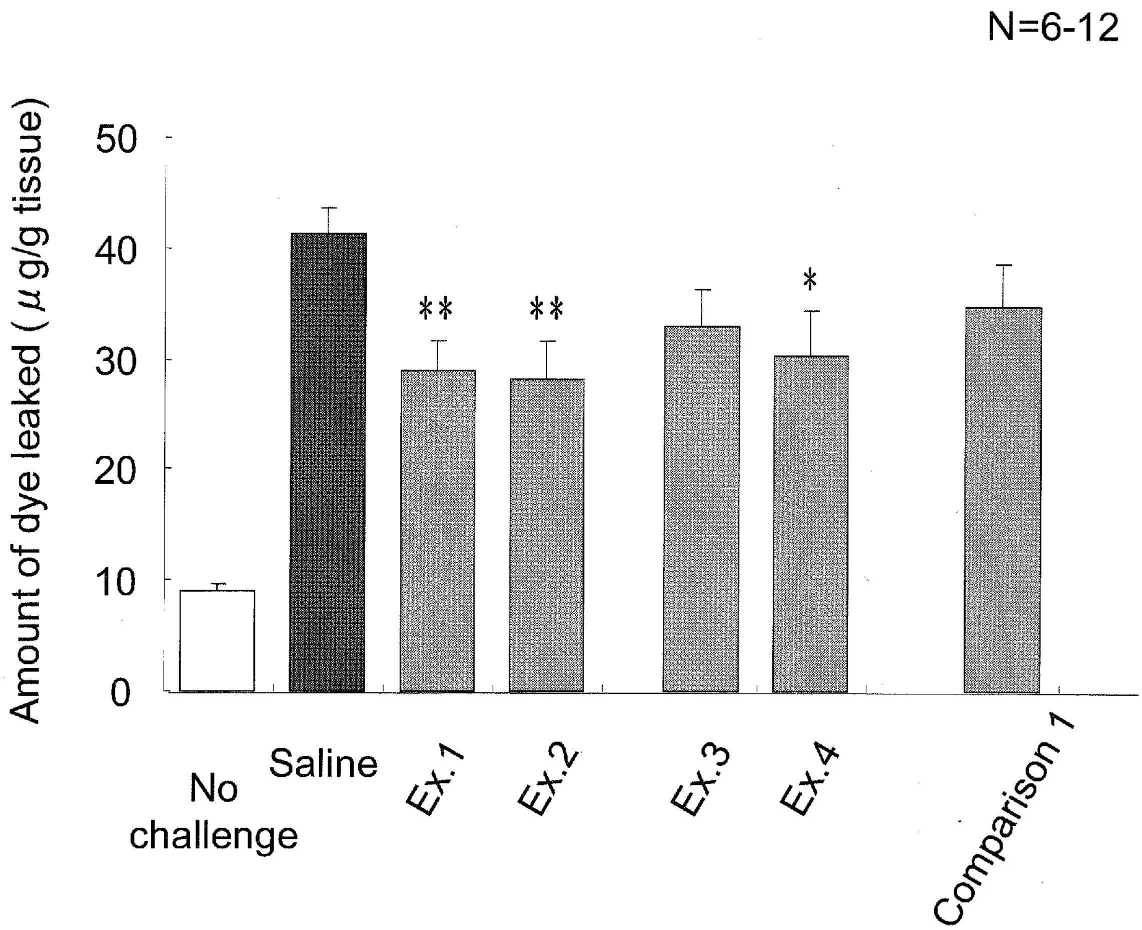
N=6-12
Amount of dye leaked (μ g/g tissue)
50
40
30
20
10
0
**
**
*
No challenge
Saline
Ex.1
Ex.2
Ex.3
Ex.4
Comparison 1

Fig. 2

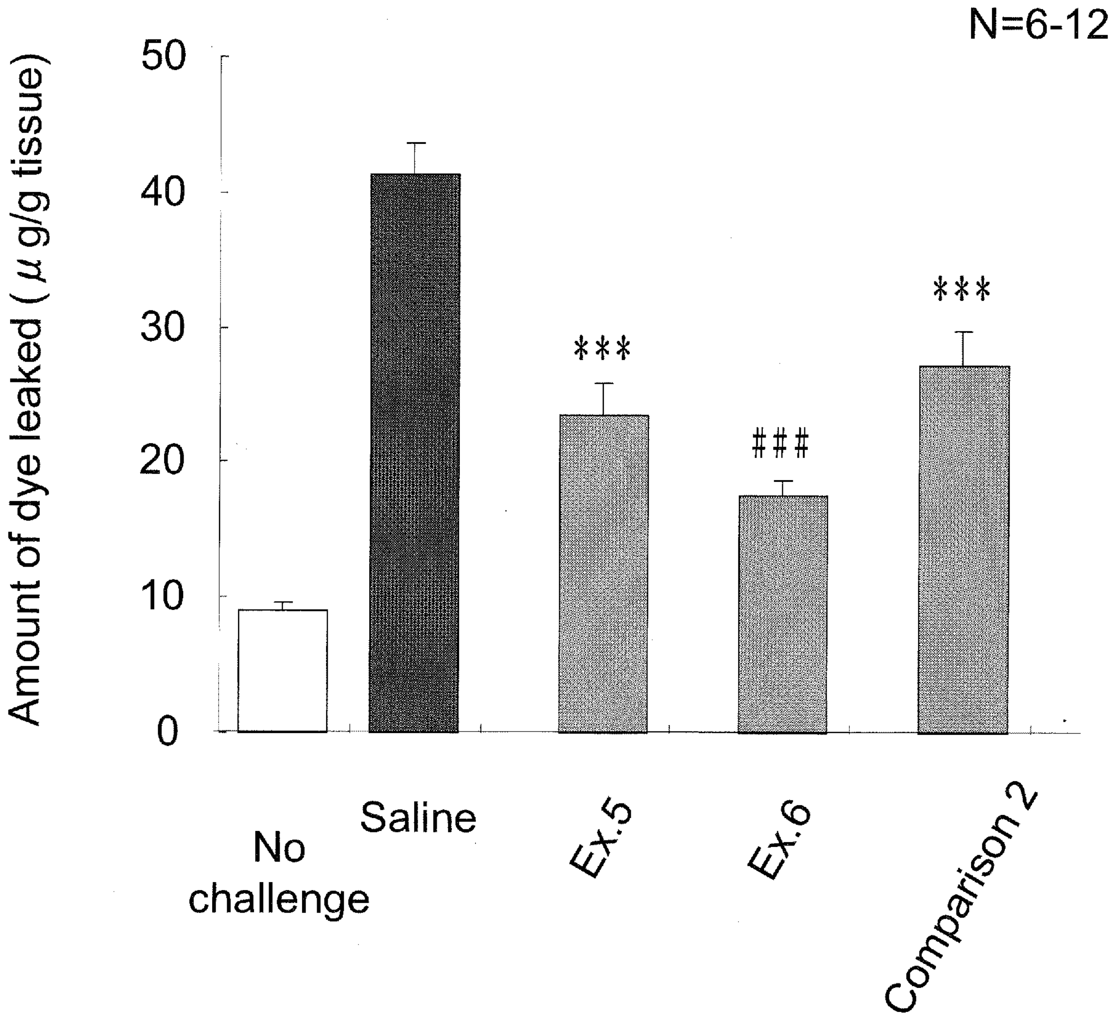
N=6-12
Amount of dye leaked ( μ g/g tissue)
50
40
30
20
10
0
***
###
***
No challenge
Saline
Ex.5
Ex.6
Comparison 2

Fig. 3

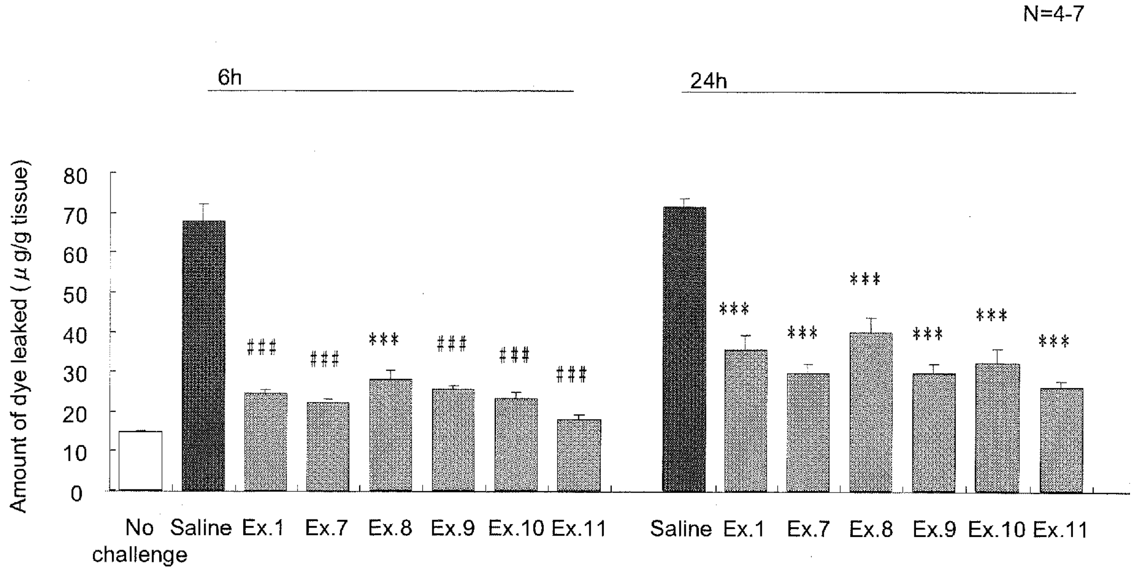
N=4-7
6h
24h
Amount of dye leaked (μ g/g tissue)
80
70
60
50
40
30
20
10
0
###
###
***
###
###
###
***
***
***
***
***
***
No challenge
Saline
Ex.1
Ex.7
Ex.8
Ex.9
Ex.10
Ex.11
Saline
Ex.1
Ex.7
Ex.8
Ex.9
Ex.10
Ex.11

Fig. 4

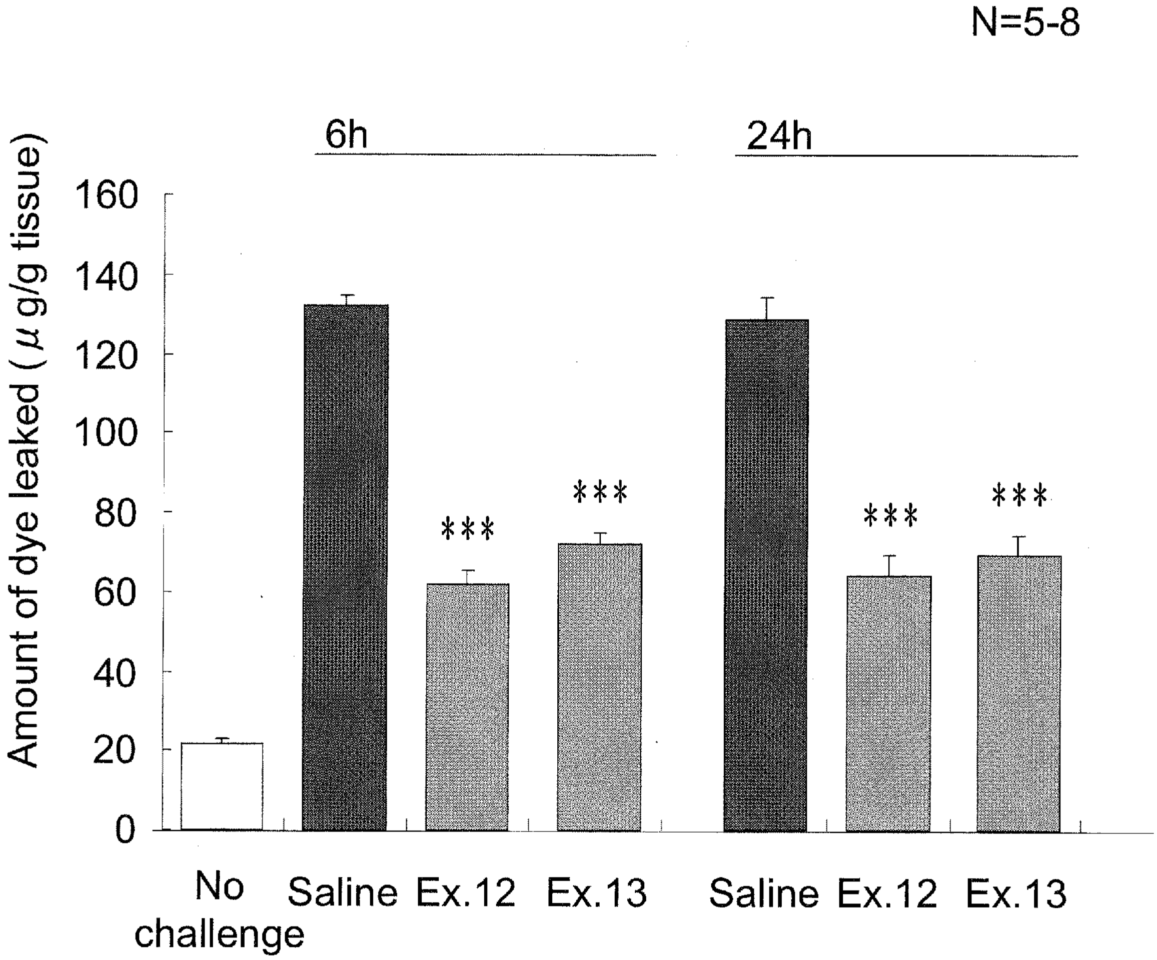
N=5-8
6h
24h
Amount of dye leaked (μg/g tissue)
160
140
120
100
80
60
40
20
0
***
***
***
***
No challenge
Saline
Ex.12
Ex.13
Saline
Ex.12
Ex.13

**INTERNATIONAL SEARCH REPORT**

International application No.
PCT/JP2008/066185

A. CLASSIFICATION OF SUBJECT MATTER
*A61K31/335*(2006.01)i, *A61K47/02*(2006.01)i, *A61K47/16*(2006.01)i, *A61K47/32*(2006.01)i, *A61P27/02*(2006.01)i, *A61P27/14*(2006.01)i

According to International Patent Classification (IPC) or to both national classification and IPC

B. FIELDS SEARCHED

Minimum documentation searched (classification system followed by classification symbols)
A61K31/335, A61K47/02, A61K47/16, A61K47/32, A61P27/02, A61P27/14

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
BIOSIS(STN), CAplus(STN), EMBASE(STN), MEDLINE(STN), REGISTRY(STN), JSTPlus(JDreamII), JMEDPlus(JDreamII), JST7580(JDreamII),

C. DOCUMENTS CONSIDERED TO BE RELEVANT

| Category* | Citation of document, with indication, where appropriate, of the relevant passages | Relevant to claim No. |
|---|---|---|
| X | JP 2004-536096 A (ALCON INC.), 02 December, 2004 (02.12.04), Claims; examples & US 2003/0055102 A1 & US 2005/0158387 A1 & US 2007/0142458 A1 & EP 1399127 A1 & WO 2003/002093 A1 | 1-8,10-17,20 |
| X | JP 9-510235 A (ALCON LAB INC.), 14 October, 1997 (14.10.97), Claims; examples & US 5641805 A & EP 799044 A2 & WO 1996/039147 A2 | 1-8,10-17,20 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>24 October, 2008 (24.10.08) | Date of mailing of the international search report<br>04 November, 2008 (04.11.08) |
| Name and mailing address of the ISA/<br>Japanese Patent Office<br>Facsimile No. | Authorized officer<br><br>Telephone No. |

Form PCT/ISA/210 (second sheet) (April 2007)

**INTERNATIONAL SEARCH REPORT**

International application No.
PCT/JP2008/066185

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | JP 2006-508138 A (ALCON INC.),<br>09 March, 2006 (09.03.06),<br>Claims; examples<br>& US 2004/0097474 A1 & EP 1560586 A1<br>& WO 2004/043470 A1 | 1-8<br>10-17,20 |
| A | WO 2005/102313 A1 (KYOWA HAKKO KOGYO KABUSHIKI KAISHA),<br>03 November, 2005 (03.11.05),<br>Par. Nos. [0020], [0027], [0028]<br>& US 2007/0225356 A1 & EP 1757285 A1 | 1-18,20 |
| A | WO 2006/009093 A1 (KYOWA HAKKO KOGYO KABUSHIKI KAISHA),<br>26 January, 2006 (26.01.06),<br>Par. Nos. [0029], [0037], [0038]<br>& EP 1769794 A1 | 1-18,20 |
| A | JP 63-10784 A (KYOWA HAKKO KOGYO KABUSHIKI KAISHA),<br>18 January, 1988 (18.01.88),<br>Claims; page 3, lower right column, 2nd and 3rd paragraphs<br>& US 5116863 A & EP 235796 A2 | 1-18,20 |

Form PCT/ISA/210 (continuation of second sheet) (April 2007)

**INTERNATIONAL SEARCH REPORT**

International application No.
PCT/JP2008/066185

Continuation of B. FIELDS SEARCHED

Electronic data base consulted during the international search (name of data base and, where practicable, search terms used)

Igaku Chuo Zasshi WEB

Form PCT/ISA/210 (extra sheet) (April 2007)

**INTERNATIONAL SEARCH REPORT**

International application No.
PCT/JP2008/066185

**Box No. II Observations where certain claims were found unsearchable (Continuation of item 2 of first sheet)**

This international search report has not been established in respect of certain claims under Article 17(2)(a) for the following reasons:

1. [x] Claims Nos.: 19
because they relate to subject matter not required to be searched by this Authority, namely:
Claim 19 pertains to a method for treatment of a human body by therapy and thus relates to a subject matter which this International Searching Authority is not required, under the provisions of PCT Article 17(2)(a)(i) and PCT Rule 39.1(iv), to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying additional fees, this Authority did not invite payment of additional fees.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the

[ ] The additional search fees were accompanied by the applicant's protest and, where applicable, payment of a protest fee.

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 63010784 A
- US 5116863 A
- JP 9510235 A
- US 5641805 A
- JP 2004536096 A
- US 6995186 B


### Non-patent literature cited in the description


- **Tamura T ; Sato H ; Miki I ; Suzuki K ; Ohmori K ; Karasawa A.** Effects of orally administered olopatadine hydrochloride on the ocular allergic reaction in rats. *Allergol Int.,* 2003, vol. 52, 77-83
- **Tamura T ; SatoH ; Miki I ; Suzuki K ; Ohmori K ; Karasawa A.** Effects of orally administered olopatadine hydrochloride on the ocular allergic reaction in rats. *Allergol Int.,* 2003, vol. 52, 77-83